Europäisches Patentamt

European Patent Office

Office européen des brevets

(11) Veröffentlichungsnummer: **0 236 997**
**A2**

(12) ## EUROPÄISCHE PATENTANMELDUNG

(21) Anmeldenummer: **87103290.0**

(22) Anmeldetag: **07.03.87**

(51) Int. Cl.³: **G 01 N 33/533**
//C12Q1/28, C12Q1/30,
G01N33/58

(30) Priorität: **14.03.86 DE 3608546**

(43) Veröffentlichungstag der Anmeldung:
**16.09.87 Patentblatt 87/38**

(84) Benannte Vertragsstaaten:
**AT BE CH DE ES FR GB GR IT LI LU NL SE**

(71) Anmelder: **Henning Berlin GmbH Chemie und Pharmawerk**
**Komturstrasse 19-20**
**D-1000 Berlin 42(DE)**

(72) Erfinder: **Gadow, André, Dr.**
**Lettbergerstrasse 43**
**1000 Berlin 47(DE)**

(74) Vertreter: **Werner, Hans-Karsten, Dr. et al,**
**Deichmannhaus am Hauptbahnhof**
**D-5000 Köln 1(DE)**

(54) **Lumineszenz-Immuno-Teste, Verfahren zur Stabilisierung und Qualitätskontrolle derselben.**

(57) Lumineszenz-Immuno-Teste bestehen aus einem zur Lumineszenz befähigten, mit Phthalhydraziden markierten Antigen oder Antikörper, einem vorzugsweise trägergebundenen Antikörper oder Antigen und einem die Lumineszenz auslösenden oxidierenden Reagenz und sind dadurch gekennzeichnet, daß das oxidierende Reagenz eine vorgefertigte, gegebenenfalls durch ein Bakteriostatikum stabilisierte Lösung von Katalase und der Initiator eine mindestens 20 Minuten alte, vorgefertigte alkalische Peroxidlösung ist. Diese Lumineszenz-Immuno-Teste sind besonders stabil sowie einfach und preiswert einer Qualitätskontrolle zu underziehen.

Abb. 1

# Lumineszenz-Immuno-Teste, Verfahren zur Stabilisierung und Qualitätskontrolle derselben

Gegenstand der vorliegenden Erfindung sind Lumineszenz-Immuno-Teste bestehend aus einem zur Lumineszenz befähigten, mit Phthalhydraziden markierten Antigen oder Antikörper, einem vorzugsweise trägergebundenen Antikörper oder Antigen und einem die Lumineszenz auslösenden oxidierenden Reagenz sowie Verfahren zur Stabilisierung und Qualitätskontrolle derartiger Teste.

Lumineszenz-Immuno-Teste sind prinzipiell gut geeignet für immunologische Bestimmungen, da sie bei sorgfältiger und kompetenter Handhabung alle Anforderungen an Präzision und zu erreichende Meßgrenzen in optimaler Weise erfüllen. Bislang ist diese Methode jedoch auf wenige spezialisierte Laboratorien beschränkt geblieben, da die Handhabung bisher noch relativ umständlich ist und nur bei sorgfältigster und kompetenter Handhabung zuverlässige Ergebnisse erzielt werden.

Ein weiterer Nachteil von Lumineszenz-Immuno-Testen besteht darin, daß die oxidierenden Reagenzien zur Auslösung der Lumineszenz nicht über längere Zeit haltbar sind und deshalb bisher stets frisch angesetzt werden mußten. Dies wiederum macht die notwendige Qualitätskontrolle sehr schwierig und aufwendig.

Die Erfindung hat sich somit zunächst einmal die Aufgabe gestellt, möglichst viele der notwendigen Reagenzien und Komponenten in eine stabile Form zu bringen, für die dann auch eine vereinfachte und zuverlässige Qualitätskontrolle möglich ist.

In der deutschen Patentanmeldung P 34 39 742 der Anmelderin ist ein Verfahren zur Initiierung der Lichtemission durch Oxidation von Phthalhydraziden in Lumineszenz-Immuno-Testen vorgeschlagen worden durch Zugabe von Pseudoperoxidase, wässrigem Natriumhydroxid und wässrigem Peroxid, bei dem die Pseudoperoxidase vor der Messung dem Testansatz zugegeben wird und die Initiierung der Lichtemission durch Zugabe einer 0,3 bis 10 Stunden alten Lösung von alkalischem Peroxid erfolgt. Versuche, die Mikroperoxidase in gelöster Form zu stabilisieren, sind gescheitert, da dieses Enzym insbesondere bei höheren Temperaturen des Versandes oder der Lagerung mehr oder weniger stark abgebaut wird und seine Aktivität verliert. Schroeder und Yeager (Analytical Chemistry, Vol. 50, No. 8, Seiten 1114-1120) haben in einer Vergleichsstudie verschiedene oxidierende Reagenzien untersucht. Aus dieser Vergleichsstudie geht hervor, daß insbesondere Hematin und Mikroperoxidase besonders geeignet sind, weshalb auch in der Folgezeit die Mikroperoxidase als der Katalysator der Wahl angesehen wurde. Leider ist es jedoch nach wie vor nötig, die Mikroperoxidase-Lösung zumindest täglich frisch anzusetzen und am selben Tage zu verbrauchen. Untersuchungen der Anmelderin führten zu dem Ergebnis, daß auch Hemin und Hematin keine lagerstabilen Lösungen geben. Versuche mit Peroxidase führten im Vergleich zur Mikroperoxidase zu deutlich schlechterer Signalausbeute, so daß auch sie für die Praxis nicht in Frage kommt.

Überraschenderweise wurde gefunden, daß das Enzym Katalase, insbesondere in gepufferter Form und in Gegenwart eines Bakteriostatikums, sehr stabile Lösungen liefert, die auch gute Lichtausbeuten bei der Verwendung als Katalysator zur Auslösung der Lumineszenz gewährleisten.

Besonders überraschend war, daß als Bakteriostatikum nicht nur Antibiotika, sondern auch Natriumazid verwendet werden kann. Da Natriumazid als Enzymgift bekannt ist, war keinesfalls vorherzusehen, daß dieses Mittel für die Stabilisierung einer Katalase-Lösung für Lumineszenz-Immuno-Teste überhaupt geeignet wäre. Als Puffer für die Katalase-Lösung eignen sich insbesondere

a) physiologische Kochsalzlösung (0,15 Mol/l NaCl),
b) 0,05 Mol/l Phosphat (pH 6),
beide gegebenenfalls unter Zusatz von 10 µMol/l Thymol.

Die neuen erfindungsgemäßen Lumineszenz-Immuno-Teste sind somit dadurch gekennzeichnet, daß das oxidierende Reagenz eine vorgefertigte, gegebenenfalls durch ein Bakteriostatikum stabilisierte Lösung von Katalase und der Initiator eine mindestens 20 Minuten alte, vorgefertigte alkalische Peroxidlösung ist. Als Peroxide haben sich insbesondere komplexierte Peroxide bewährt, da diese in fester Form lagerbeständig sind und in alkalischer Lösung nach mindestens 20 Minuten Lagerung für die Dauer eines Meßtages ausreichend stabil sind. Das Alter von 20 Minuten ist erforderlich, da während der ersten 20 Minuten nach dem Zusammengeben von Peroxid und Alkali eine deutliche Signalverstärkung im Lumineszenz-Immuno-Test beobachtet wird, die zu einer wesentlichen Verbesserung der Empfindlichkeit und der unteren Nachweisgrenze führt. Während der ersten 20 Minuten führt diese Reifung der Lösung jedoch zu Verfälschungen der Meßwerte. Optimale Ergebnisse werden somit erzielt, wenn die alkalischen Peroxidlösungen 20 Minuten bis 10 Stunden alt sind.

Erfindungsgemäß ist es jetzt möglich, Lumineszenz-Immuno-Teste bestehend aus einem zur Lumineszenz befähigten, mit Phthalhydraziden markierten Antigen oder Antikörper, einem vorzugsweise trägergebundenen Antikörper oder Antigen und einem die Lumineszenz auslösenden oxidierenden Reagenz zu stabilisieren, dadurch daß das oxidierende Reagenz in Form einer vorgefertigten, gegebenenfalls durch ein Bakteriostatikum stabilisierten Lösung von Katalase und der Initiator in Form einer mindestens 20 Minuten alten, vorgefertigten alkalischen Peroxidlösung zugegeben werden.

Als Peroxide eignen sich insbesondere die festen, komplexierten Peroxide wie Natriumperoxid, Natriumpercarbonat, Natriumperborat, Ammoniumperoxodisulfat und handelsübliche Perhydrit[R]-Tabletten, in denen Wasserstoffperoxid an Harnstoff gebunden ist.

Als Phthalhydrazide eignen sich insbesondere das 6/N-(4-Aminobutyl-N-ethyl)isoluminol7-hemisuccinamid (ABEI-H) sowie Substanzen wie sie beschrieben sind in den US-PS 4,363,759, 4,334,069, 4,297,273, 4,225,485 und 4,331,808. Weitere gut geeignete Phthalhydrazide sind solche mit einem anellierten Benzolring wie die Naphthylhydrazide.

Erfindungsgemäß ist es jetzt auch wesentlich einfacher und schneller möglich, die erforderliche Qualitätskontrolle der Lumineszenz-Immuno-Teste durchzuführen. Diese Qualitätskontrolle sollte sowohl den einwandfreien Zustand der die Lumineszenz auslösenden Reagenzien als auch des Meßgerätes erfassen. Dabei hat sich gezeigt, daß die unterschiedlichen Konzepte der heute handelsüblichen Luminometer zu erheblichen Abweichungen und dadurch nicht akzeptablen Störungen führen können.

Je nach der Art der Messung der Chemilumineszenz-Signale erhält man nämlich sehr unterschiedliche Meßgrößen. So erfassen einige Luminometer die Chemilumineszenz-Signale in Form von Photonenströmen (Meßgröße = Volt) und andere in Form von Einzelphotonen-Ereignissen (Meßgröße = Impulse oder Counts). Weiterhin hat sich gezeigt, daß selbst bei Verwendung mehrere Geräte des gleichen Gerätetyps unter Verwendung ansonsten völlig gleicher Reagenzien von Gerät zu Gerät Schwankungen von mehr als 20% beobachtet werden. Dies liegt offensichtlich an der Schwierigkeit einer Eichung der in Luminometern verwendeten Photomultiplier.

Bei den vergleichenden Untersuchungen nicht nur verschiedener Reagenzien, sondern auch verschiedener Geräte wurde die überraschende Feststellung gemacht, daß bei der Messung von definierten chemilumineszenten Kontrollproben, die sich in ihren Meßwerten um einen definierten Faktor zwischen 2 und 100, vorzugsweise zwischen 5 und 20, unterscheiden, der Quotient aus den Meßwerten dieser Kontrollproben, unabhängig vom verwendeten Meßgerät, in relativ engem Rahmen konstant ist. Daraus ergibt sich, daß man bereits mit Hilfe von zwei Kontrollwerten I und II, die sich um einen definierten Faktor (beispielsweise 10) unterscheiden, die Geräte und Chemikalien zuverlässig und einfach prüfen kann, sofern folgende Werte gemessen bzw. errechnet werden:

a) Geräteleerwert (GLW)
b) Reagenzienleerwert (RLW)
c) Kontrollwerte I und II (KI und KII)
d) der Quotient KII/KI und
e) der Quotient KI/RLW.

In einigen Fällen kann es sinnvoll sein, zusätzlich den Quotienten RLW/GLW zu errechnen.

Auf alle Fälle ist es möglich, diesen Meßwert einem Rechner einzugeben, der bei Abweichungen von den Sollwerten die möglichen Fehlerquellen direkt anzeigt.

Besonders überraschend war der Befund, daß außer den oben genannten Meßwerten und Quotienten die nur vor der Bestimmungsserie ermittelt werden können, auch während der Bestimmungsserie die Qualitätskontrolle fortgesetzt werden kann, da auch bei den eigentlichen Messungen die Signal-Halbwertzeit T 1/2 nahezu konstant ist.

Durch Messung dieser Signal-Halbwertzeit und Vergleich mit dem ursprünglichen Wert unmittelbar nach der Kalibrierung des Gerätes wird angezeigt, daß noch alle Parameter in Ordnung sind und somit zuverlässige Ergebnisse erwartet werden können. Ein allmähliches oder plötzliches Abweichen der Signal-Halbwertzeit zeigt an, daß entweder am Gerät oder den Reagenzien Fehler eingetreten sind, die die Zuverlässigkeit des Endergebnisses beeinträchtigen können.

Der Geräteleerwert (GLW) wird ermittelt durch eine Messung ohne Zusatz von Reagenzien. Die erhaltenen Werte sollten hier relativ klein sein und den vom Gerätehersteller angegebenen Erfahrungswerten entsprechen. Eine Störung durch unerwünschtes Fremdlicht äußert sich durch extrem hohe Impulsraten, oft über der oberen Erfassungsgrenze des Gerätes.

Der Reagenzienleerwert (RLW) wird ermittelt, indem vorab die Katalase-Lösung und in der Meßposition des Luminometers die alkalische Peroxidlösung in ein Meßröhrchen injiziert werden und die Lumineszenz gemessen wird.

Diese Meßwerte liegen im allgemeinen höher als der GLW, dürfen aber wiederum eine aus Erfahrungswerten festzusetzende obere Grenze nicht überschreiten. Ein deutliches Überschreiten des Grenzwertes RLW bei korrektem GLW deutet auf die Kontamination eines oder beider chemilumineszenz-auslösenden Reagenzien mit einer Substanz hin, die ein entsprechendes unspezifisches Chemilumineszenz-Signal erzeugt. Auch kontaminierte Meßröhrchen können die Ursache sein. Ist jedoch GLW = RLW, so kann dies an der Verwendung bereits unbrauchbar gewordener Reagenzien liegen oder an einem fehlerhaften Injektionssystem.

Der Quotient KII/KI sollte dem Wert entsprechen, auf den hin die Kontroll-Lösungen KI und KII eingestellt wurden. Der Quotient KI/RLW sollte im Bereich zwischen 20 und 70 liegen. Um eine ausreichende Zuverlässigkeit zu gewährleisten, sollte jede Messung mindestens in Doppel-, besser in Dreifach-Bestimmungen erfolgen und der Mittelwert hieraus verwertet werden. Liegt der Variationskoeffizient dieser Mehrfach-Bestimmungen über 5%, so ist nach der Ursache zu suchen. Bei deutlichen Abweichungen der Quotienten KII/KI und KI/RLW deuten Höhe und Art der Abweichung wiederum auf bestimmte Fehler hin, nach denen gezielt gesucht werden kann.

Durch die Tatsache, daß bei einwandfreiem Gerät und einwandfreien Reagenzien die Signal-Halbwertzeit T 1/2 nahezu konstant ist, ermöglicht die Messung dieses Parameters eine ständige Überprüfung jedes einzelnen Meßwertes. Es hat sich weiterhin gezeigt, daß die Messung bzw. Berechnung der oben genannten Meßwerte und Quotienten sowie insbesondere der Signal-Halbwertzeit nicht nur für die erfindungsgemäßen Lumineszenz-Immuno-Teste anwendbar ist, sondern in gleicher Weise auch für ande-- re Teste, in denen als lumineszierendes System mit Acridingruppen markierte Antigene oder Antikörper verwendet werden. Als Acridin-Derivate eignen sich insbesondere Substanzen, wie sie beschreiben sind in der EP-OS 0 082 636.

Ein weiterer Gegenstand der vorliegenden Erfindung ist somit auch das Verfahren zur Qualitätskontrolle von Lumineszenz-Immuno-Testen bestehend aus einem zur Lumineszenz befähigten, mit Phthalhydraziden oder Acridin-Gruppen markierten Antigen oder Antikörper, einem vorzugsweise trägergebundenen Antikörper oder Antigen und einem die Lumineszenz auslösenden oxidierenden Reagenz, dadurch gekennzeichnet, daß erforderlichenfalls das oxidierende Reagenz eine vorgefertigte, gegebenenfalls durch ein Bakteriostatikum stabilisierte Lösung von Katalase und der Initiator eine mindestens 20 Minuten alte, vorgefertigte alkalische Peroxidlösung ist und daß folgende Werte gemessen bzw. errechnet werden:

a) Geräteleerwert (GLW)

b) Reagenzienleerwert (RLW)

c) Kontrollwerte I und II (KI und KII)

d) der Quotient KII/KI und

e) der Quotient KI/RLW.

Vorzugsweise werden die Meßwerte a) bis e) in einen Rechner eingegeben, der bei Abweichungen von Sollwerten die möglichen Fehlerquellen anzeigt. Vorzugsweise wird zusätzlich die Signal-Halbwertzeit T 1/2 gemessen und mit dem Sollwert verglichen und damit eine permanente Qualitätskontrolle erzielt.

In Sonderfällen und bei bestimmten Gerätetypen mag es sinnvoll sein, zusätzlich den Quotienten RLW/GLW zu errechnen, um bei Abweichungen dieses Quotienten auf bestimmte Fehlermöglichkeiten hinzuweisen.

Die erfindungsgemäßen Lumineszenz-Immuno-Teste, das Verfahren zu ihrer Stabilisierung und das Verfahren zur Qualitätskontrolle sind in den nachfolgenden Beispielen näher erläutert.

B e i s p i e l 1

Herstellung einer gebrauchsfertigen und lagerstabilen Katalase-Lösung:

$4 - 20 \times 10^6$ Units Katalase aus Rinderleber, Molekulargewicht ca. 240.000, werden in 1 Liter 0,15 mol/l NaCl und 0,1% (Gew./Vol.) Natriumazid gelöst. Ein Unit Katalase ist dabei definitionsgemäß die Enzymmenge, die 1 µmol Wasserstoffperoxid pro Minute bei einem pH-Wert von 7,0 und einer Temperatur von 25°C umsetzt. Die zu wählende Endkonzentration in Units Katalase/l hängt von der Beschaffenheit des für die Auslösung des Chemilumineszenz-Signals von Phthalhydraziden und deren Derivaten zusätzlich erforderlichen bzw. verwendeten Reagenz ab und ist entsprechend anzupassen bzw. zu optimieren.

Die oben beschriebene Lösung ist bei 4°C 6 Monate und bei Raumtemperatur 2 Monate stabil. Selbst bei 37°C weist sie noch eine Haltbarkeit von 35 Tagen auf.

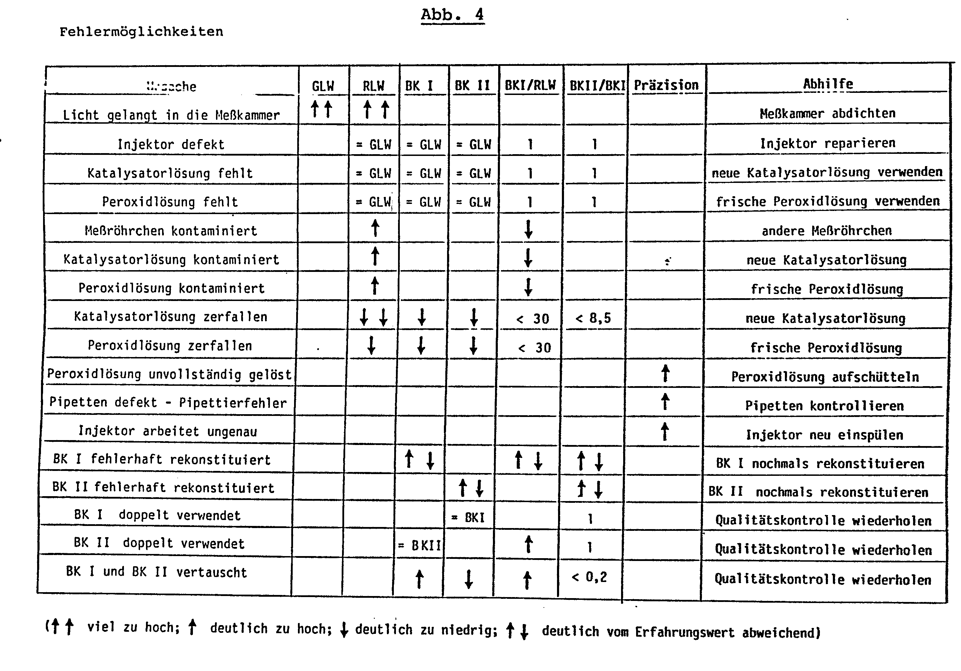

Die gleiche Katalase-Lösung ohne Zusatz von Natriumazid ist, insbesondere bei höheren Temperaturen, sogar noch etwas besser haltbar, jedoch besteht die Gefahr einer bakteriellen Infektion. Die Meßwerte für die Langzeitstabilität erfindungsgemäßer Katalase-Lösungen finden sich in der anliegenden Abbildung 2.

Aus den Ergebnissen ergibt sich, daß diese Lösung auch in den Sommermonaten ohne weiteres vom Hersteller zum Anwender versendet werden kann, ohne daß zu erwarten ist, daß die Lösung unbrauchbar wird. Durch den Zusatz von Natriumazid wird selbst nach 6 Monaten kein bakterielles Wachstum beobachtet. Die Enzymaktivität wird durch diesen Zusatz nur unwesentlich beeinträchtigt.

Die Meßwerte der Abbildung 2 wurden erhalten, indem jeweils 100 µl einer Kontrollprobe (Phthalhydrazid-Derivat markiertes Protein), die in einem Berthold-Luminometer LB 9500 ein definiertes Chemilumineszenz-Signal erzeugen kann, mit 300 µl einer Katalase-Gebrauchslösung versetzt wurden, die unter den angegebenen Bedingungen gelagert wurde. Nach Einbringen der Probe in die Meßposition wurde durch Injektion von 300 µl alkalischer Peroxidlösung die Chemilumineszenz-Reaktion gestartet. Die erhaltenen Signale wurden denen mit einer frisch hergestellten Katalase-Gebrauchslösung gegenübergestellt und in Prozent angegeben.

B e i s p i e l 2

Herstellung von alkalischen Peroxidlösungen:

Lösung A

100 mg Natriumpercarbonat ($2Na_2CO_3$ x $3H_2O_2$) werden in 100 ml 0,2 N Natronlauge gelöst. Diese Lösung bleibt vor der Anwendung 30 Minuten bei Raumtemperatur stehen. Zur Auslösung der Chemilumineszenz-Signale von Phthalhydrazid-Derivaten und Acridinium-Derivaten werden 300 µl dieser Lösung in der Meßposition des Luminometers in geeignete Meßröhrchen injiziert, die die zu messende Probe enthalten.

Die Lösung ist bei Raumtemperatur (18 bis 22°C) mindestens einen Meßtag und bei Kühlschranktemperatur (4°C) ca. 7 Tage stabil. 100 mg Natriumpercarbonat enthalten so viel Aktivsauerstoff wie ca. 80 mg 35% Wasserstoffperoxid.

Lösung B

200 mg Natriumperborat ($NaBO_2$ x $H_2O_2$ x $3H_2O$) werden in 100 ml 0,2 N Natronlauge gelöst. Verwendung wie bei Lösung A. Lösung B ist bei Raumtemperatur (18 bis 22°C) mindestens einen Meßtag und bei Kühlschranktemperatur (4°C) mindestens 10 Tage stabil. 100 mg Natriumperborat enthalten so viel Aktivsauerstoff wie ca. 61 mg 35% Wasserstoffperoxid.

Lösung C

1 Perhydrit[(R)]-Tablette (= 1 g) wird gelöst in 100 ml 1 N Natronlauge. Verwendung wie bei Lösung A. Lösung C ist bei Raumtemperatur (18 bis 22°C) mindestens einen Meßtag und bei Kühlschranktemperatur (4°C) mindestens 10 Tage stabil. 1 Perhydrit[(R)]-Tablette (= 1 g) enthält so viel Aktivsauerstoff wie ca. 1 g 35% Wasserstoffperoxid.

B e i s p i e l   3

Lumineszenz-Immuno-Test-Kit:

Für die Lumineszenz-Immuno-Teste werden zwei getrennte Packungen mit folgendem Inhalt hergestellt:
Immuno-Kit (ausreichend für 100 Bestimmungen):
In separaten Flaschen werden für 100 Bestimmungen ausreichende Mengen trägergebundener Antikörper oder Antigene sowie mit einem Phthalhydrazid-Derivat markierte Antikörper bzw. Antigene abgepackt sowie eine Standard-Verdünnungsreihe mit definierten Konzentrationen des Antigens bzw. Antikörpers zum Kalibrieren. Bei Immuno-Testen z.B. nach einer Sandwich-Methode wird ein entsprechender zweiter Antigen- oder speziesspezifischer Antikörper mitgeliefert. Diese Immuno-Kits werden für die jeweilige Bestimmung zusammengestellt, sind jedoch allesamt einsetzbar zusammen mit dem nachfolgenden Reagenzien-Kit:

Reagenzien-Kit (für 1.000 bis 5.000 Bestimmungen):
Im Reagenzien-Kit sind zusammengestellt die mit Azid stabilisierte Katalase-Lösung, festes, komplexiertes Peroxid und 1 N Natronlauge sowie Kontroll-Lösungen I und II mit dem lumineszierenden Derivat.

Zur Anwendung wird das Peroxid in der Natronlauge gelöst. Diese Lösung ist nach 20 Minuten Alterung einsetzbar und mindestens während des ganzen Meßtages haltbar.

**B e i s p i e l 4**

Qualitätskontrolle:

Über eine Reihe von verschiedenen Luminometern wurden die Geräteleerwerte (GLW), die Reagenzienleerwerte (RLW) und die Kontrollwerte I und II (KI und KII) gemessen und die Quotienten KII/KI und KI/RLW berechnet. Die Auslösung der Chemilumineszenz-Signale erfolgte mit der oben beschriebenen Katalase-Lösung und alkalischer Peroxidlösung. Jeder Wert wurde dreifach gemessen und der Mittelwert eingesetzt. Die Variationskoeffizienten lagen stets unter 5%.

Die Meßergebnisse sind in der nachfolgenden Tabelle zusammengestellt.

| Luminometer-Gerätetyp | Meßdauer | RLW | K I | K II | KII/KI | KI/RLW |
|---|---|---|---|---|---|---|
| LKB 1251 | 20 sec. | 10 (mV) | 349 | 3079 | 8.82 | 34.9 |
| Berthold LB 950 | 10 sec. | 12 (Imp. x 10⁻³) | 540 | 5350 | 9.9 | 45 |
| Berthold LB 9500, Gerät 1 | 10 sec. | 664 (Impulse) | 22516 | 214070 | 9.51 | 33.9 |
| LB 9500,Gerät 2 | 10 sec. | 899 (Impulse) | 31352 | 298094 | 9.5 | 34.9 |
| LB 9500,Gerät 3 | 10 sec. | 1314 (Impulse) | 39784 | 406095 | 10.2 | 30.2 |
| LB 9500,Gerät 4 | 10 sec. | 620 (Impulse) | 21376 | 218080 | 10.2 | 34.5 |

Um die Auswirkung typischer Fehler an den Reagenzien zu kontrollieren, wurden diese bewußt herbeigeführt. Dazu wurde unter Verwendung des Luminometers Berthold LB 9500 und unter Verwendung der oben beschriebenen erfindungsgemäß hergestellten bzw. bewußt fehlerhaften modifizierten Reagenzien die folgenden Werte gemessen bzw. errechnet:

| | RLW (Impulse) | K I (Impulse) | K II (Impulse) | K II/K I | K I/RLW |
|---|---|---|---|---|---|
| Geräteleerwert (GLW)-Mittel = 40 Impulse | | | | | |
| intakte Referenz | 640 | 36270 | 367116 | 10.1 | 56.67 |
| $H_2O$ anstatt Natronlauge | 70 | 61 | 64 | 1.05 | 0.86 |
| Peroxid vergessen | 62 | 61 | 66 | 1.08 | 1.06 |
| Katalysator vergessen | 81 | 96 | 106 | 1.19 | 1.31 |
| Katalysator 10fach verdünnt | 83 | 7117 | 66818 | 9.39 | 85.75 |
| Katalysator 100fach verdünnt | 68 | 980 | 6739 | 6.88 | 14.41 |
| alk. Peroxidlsg. zu 50% zerfallen | 655 | 27300 | 264400 | 9.68 | 41.68 |
| alk. Peroxidlsg. zu 95% zerfallen | 430 | 6380 | 58162 | 9.12 | 14.84 |

Ein typisches Beispiel für den Computer-Ergebnisausdruck eines erfindungsgemäß durchgeführten Qualitätskontrollablaufes zur Prüfung des Meßgerätes und der erfindungsgemäß hergestellten Katalase- und alkalischen Peroxid-Gebrauchslösungen sieht folgendermaßen aus:

```
          * * * * * * * * *
          Qualitätskontrolle
               Datum
          * * * * * * * * *

          GLW1 37
          GLW2 41
          GLW     39

          RLW1 673
          RLW2 660
          RLW3 657
          RLW4 668
          RLW     664.5

          Kontrolle I 1 23209
          Kontrolle I 2 22381
          Kontrolle I 3 21995

          Kontrolle II 1 220195
          Kontrolle II 2 210722
          Kontrolle II 3 211293

          Kontrolle I 22516
          Kontrolle II 214070

          Präzision I 2.8 %
          Präzision II 2.5 %

          K II/K I 9.51
          K I/RLW 33.9
```

Meßsystem und Gerät in Ordnung

Ein typisches Beispiel für ein Fließdiagramm zum Ablauf eines Qualitätskontrollprogramms zur Prüfung des Meßgerätes sowie der erfindungsgemäß hergestellten Katalase- und alkalischen Peroxid-Gebrauchslösungen ist in der anliegenden Abbildung 3 dargestellt.

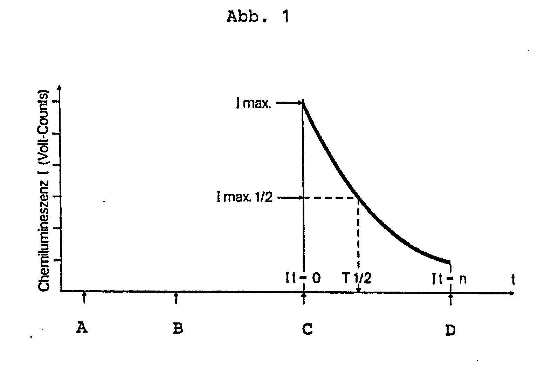

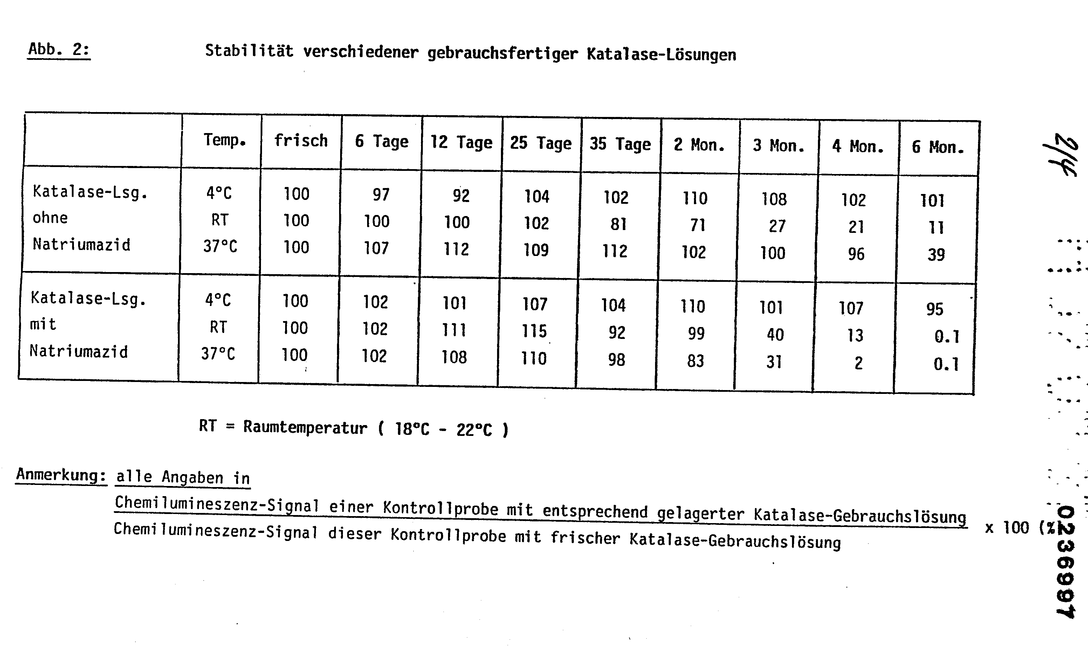

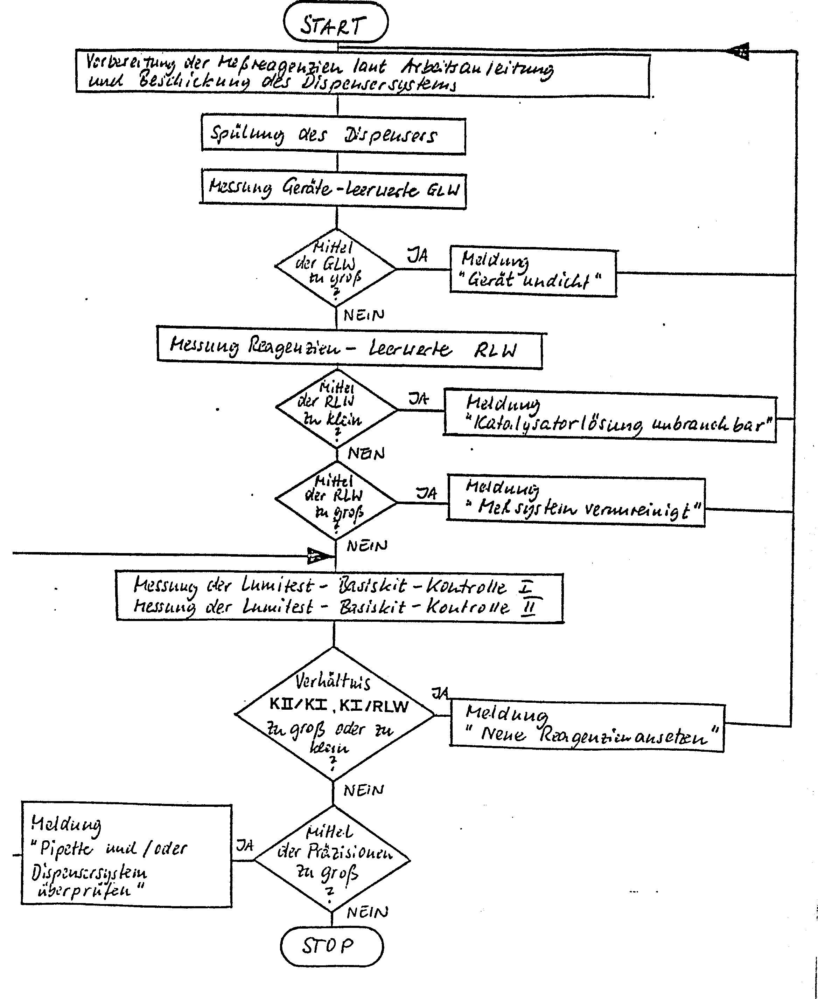

Der zeitliche Ablauf einer Chemilumineszenz-Messung unter Verwendung der erfindungsgemäß hergestellten Reagenzien ist in der anliegenden Abbildung 1 dargestellt. Nach Zugabe des Katalysators (= Katalase-Gebrauchslösung) zum Zeitpunkt A zu der zu messenden Probe (entfällt beispielsweise bei Verwendung von Acridin-Derivaten) wird das Meßröhrchen mit der Probe in das Luminometer eingebracht (Zeitpunkt B). Durch ein geeignetes Injektionssystem wird in der Meßposition gebrauchsfertige alkalische Peroxidlösung injiziert (Zeitpunkt C) und die Chemilumineszenz-Messung gestartet. Als Meßgröße dient das im Computer ermittelte Intergral unter der Kurve der Lichtintensität von t = 0 bis t = n (n = Meßdauer) zum Zeitpunkt D. Die Signal-Halbwertzeit T 1/2 ergibt sich aus dem Zeitpunkt der Erreichung der Hälfte der maximalen Intensität I max. Dieser Wert ist bei einwandfreien Geräten und einwandfreien Reagenzien über den gesamten Tagesverlauf nahezu konstant und ist somit ein Hinweis auf das weiterhin voll funktionstüchtige System.

Die umfangreichen Messungen mit dem erfindungsgemäßen System haben gezeigt, daß die Stabilität der Katalaselösung noch verbessert werden kann durch den Zusatz von Ethanol und EDTA. Durch diese Zusätze kann der beobachtete langsame Shift von höheren zu niedrigeren Werten innerhalb von Stunden verhindert werden. Ethanol wirkt als sehr langsam reagierendes Substrat der Katalase, reagiert jedoch wesentlich langsamer als Peroxid.

In Abwesenheit von Peroxid wird etwas Ethanol durch Katalase und Luftsauerstoff langsam oxidiert, ohne daß die chemilumineszenten Eigenschaften der Phthalhydrazide beeinflußt werden. EDTA wirkt komplexierend auf zweiwertige Metallionen, die ebenfalls langsam die Chemilumineszenz der Phthalhydrazide auslösen können.

Eine optimierte Katalaselösung hat daher folgende Zusammensetzung:

$4 - 20 \times 10^6$ Units Katalase aus Rinderleber, Molekulargewicht ca. 240.000, werden in 1 l 0,15 mol/l NaCl und 0,1% (Gew./Vol.) Natriumazid gelöst, wobei Ethanol (1- bis 10%-ig, vorzugsweise 3%-ig) und EDTA-Natriumsalz (Ethylendiamintetraacetat) 0,1 - 5 g/l, vorzugsweise 1 g/l zugesetzt werden.

In der Abbildung 4 ist eine Tabelle von Fehlermöglichkeiten und ihre Beseitung zusammengestellt.

0236997

Patentansprüche

1. Lumineszenz-Immuno-Teste bestehend aus einem zur Lumineszenz befähigten, mit Phthalhydraziden markierten Antigen oder Antikörper, einem vorzugsweise trägergebundenen Antikörper oder Antigen und einem die Lumineszenz auslösenden oxidierenden Reagenz, dadurch gekennzeichnet, daß das oxidierende Reagenz eine vorgefertigte, gegebenenfalls durch ein Bakteriostatikum stabilisierte Lösung von Katalase und der Initiator eine mindestens 20 Minuten alte, vorgefertigte alkalische Peroxidlösung ist.

2. Lumineszenz-Immuno-Teste gemäß Anspruch 1, dadurch gekennzeichnet, daß als Bakteriostatikum Natriumazid verwendet wird.

3. Lumineszenz-Immuno-Teste gemäß Anspruch 1 oder 2, dadurch gekennzeichnet, daß als Peroxid komplexierte Peroxide verwendet werden.

4. Lumineszenz-Immuno-Teste gemäß Anspruch 1 oder 2, dadurch gekennzeichnet, daß die Lösung zusätzlich Ethanol und EDTA enthält.

5. Verfahren zur Stabilisierung von Lumineszenz-Immuno-Testen, bestehend aus einem zur Lumineszenz befähigten, mit Phthalhydraziden markierten Antigen oder Antikörper, einem vorzugsweise trägergebundenen Antikörper oder Antigen und einem die Lumineszenz auslösenden oxidierenden Reagenz, dadurch gekennzeichnet, daß das oxidierende Reagenz in Form einer vorgefertigten, gegebenenfalls durch ein Bakteriostatikum stabilisierten Lösung von Katalase und der Initiator in Form einer mindestens 20 Minuten alten, vorgefertigten alkalischen Peroxidlösung zugegeben werden.

6. Verfahren gemäß Anspruch 5, dadurch gekennzeichnet, daß als Bakteriostatikum Natriumazid verwendet wird.

7. Verfahren gemäß Anspruch 5 oder 6, dadurch gekennzeichnet, daß als Peroxid komplexierte Peroxide verwendet werden.

8. Verfahren gemäß Anspruch 5 oder 6, dadurch gekennzeichnet, daß die Lösung zusätzlich Ethanol und EDTA enthält.

9. Verfahren zur Qualitätskontrolle von Lumineszenz-Immuno-Testen, bestehend aus einem zur Lumineszenz befähigten, mit Phthalhydraziden oder Acridin-Gruppen markierten Antigen oder Antikörper, vorzugsweise trägergebundenen Antikörpern oder Antigenen und einem die Lumineszenz auslösenden oxidierenden Reagenz, dadurch gekennzeichnet, daß erforderlichenfalls das oxidierende Reagenz eine vorgefertigte, gegebenenfalls durch ein Bakteriostatikum stabilisierte Lösung von Katalase und der Initiator eine mindestens 20 Minuten alte, vorgefertigte alkalische Peroxidlösung ist und daß folgende Werte gemessen bzw. errechnet werden:
   a) Geräteleerwert (GLW)
   b) Reagenzienleerwert (RLW)
   c) Kontrollwerte I und II (KI und KII)
   d) der Quotient KII/KI und
   e) der Quotient KI/RLW.

10. Verfahren zur Qualitätskontrolle gemäß Anspruch 9, dadurch gekennzeichnet, daß die Meßwerte a) bis e) einem Rechner eingegeben werden, der bei Abweichungen von Sollwerten die möglichen Fehlerquellen anzeigt.

11. Verfahren gemäß Anspruch 9 oder 10, dadurch gekennzeichnet, daß zusätzlich der Quotient RLW/GLW berechnet wird.

12. Verfahren nach einem der Ansprüche 9 bis 11, dadurch gekennzeichnet, daß zusätzlich die Signal-Halbwertzeit T 1/2 gemessen und mit dem Sollwert verglichen wird.

114

Abb. 1

<u>Abb. 2:</u>   Stabilität verschiedener gebrauchsfertiger Katalase-Lösungen

| | Temp. | frisch | 6 Tage | 12 Tage | 25 Tage | 35 Tage | 2 Mon. | 3 Mon. | 4 Mon. | 6 Mon. |
|---|---|---|---|---|---|---|---|---|---|---|
| Katalase-Lsg. | 4°C | 100 | 97 | 92 | 104 | 102 | 110 | 108 | 102 | 101 |
| ohne | RT | 100 | 100 | 100 | 102 | 81 | 71 | 27 | 21 | 11 |
| Natriumazid | 37°C | 100 | 107 | 112 | 109 | 112 | 102 | 100 | 96 | 39 |
| Katalase-Lsg. | 4°C | 100 | 102 | 101 | 107 | 104 | 110 | 101 | 107 | 95 |
| mit | RT | 100 | 102 | 111 | 115 | 92 | 99 | 40 | 13 | 0.1 |
| Natriumazid | 37°C | 100 | 102 | 108 | 110 | 98 | 83 | 31 | 2 | 0.1 |

RT = Raumtemperatur ( 18°C - 22°C )

<u>Anmerkung:</u> <u>alle Angaben in</u>

$$\frac{\text{Chemilumineszenz-Signal einer Kontrollprobe mit entsprechend gelagerter Katalase-Gebrauchslösung}}{\text{Chemilumineszenz-Signal dieser Kontrollprobe mit frischer Katalase-Gebrauchslösung}} \times 100 \ (\%)$$

0236997

```
                        ( START )

┌─────────────────────────────────────────────┐
│ Vorbereitung der Meßreagenzien laut Arbeitsanleitung │
│ und Beschickung des Dispensersystems                 │
└─────────────────────────────────────────────┘

        ┌────────────────────────────┐
        │ Spülung des Dispensers      │
        └────────────────────────────┘

        ┌────────────────────────────┐
        │ Messung Geräte-Leerwerte GLW │
        └────────────────────────────┘

            ◇ Mittel der GLW zu groß ?    ── JA ──►  ┌─────────────────────┐
                        │                             │ Meldung             │
                       NEIN                           │ "Gerät undicht"     │
                        │                             └─────────────────────┘

        ┌────────────────────────────────────┐
        │ Messung Reagenzien - Leerwerte RLW   │
        └────────────────────────────────────┘

            ◇ Mittel der RLW zu klein ?  ── JA ──►  ┌────────────────────────────────────┐
                        │                            │ Meldung                            │
                       NEIN                          │ "Katalysatorlösung unbrauchbar"    │
                        │                            └────────────────────────────────────┘

            ◇ Mittel der RLW zu groß ?   ── JA ──►  ┌────────────────────────────────────┐
                        │                            │ Meldung                            │
                       NEIN                          │ "Meßsystem verunreinigt"           │
                        │                            └────────────────────────────────────┘

        ┌─────────────────────────────────────────────────┐
        │ Messung der Lumitest-Basiskit-Kontrolle I         │
        │ Messung der Lumitest-Basiskit-Kontrolle II        │
        └─────────────────────────────────────────────────┘

            ◇ Verhältnis KII/KI, KI/RLW zu groß oder zu klein ? ── JA ──►  ┌──────────────────────────┐
                        │                                                   │ Meldung                  │
                       NEIN                                                 │ "Neue Reagenzien ansetzen" │
                        │                                                   └──────────────────────────┘

┌─────────────────────┐
│ Meldung             │ ◄── JA ── ◇ Mittel der Präzisionen zu groß ?
│ "Pipette und/oder   │                        │
│ Dispensersystem     │                       NEIN
│ überprüfen"         │                        │
└─────────────────────┘                    ( STOP )
```

## Abb. 4

Fehlermöglichkeiten

| Ursache | GLW | RLW | BK I | BK II | BKI/RLW | BKII/BKI | Präzision | Abhilfe |
|---|---|---|---|---|---|---|---|---|
| Licht gelangt in die Meßkammer | ↑↑ | ↑↑ | | | | | | Meßkammer abdichten |
| Injektor defekt | | = GLW | = GLW | = GLW | 1 | 1 | | Injektor reparieren |
| Katalysatorlösung fehlt | | = GLW | = GLW | = GLW | 1 | 1 | | neue Katalysatorlösung verwenden |
| Peroxidlösung fehlt | | = GLW | = GLW | = GLW | 1 | 1 | | frische Peroxidlösung verwenden |
| Meßröhrchen kontaminiert | | ↑ | | | ↓ | | | andere Meßröhrchen |
| Katalysatorlösung kontaminiert | | ↑ | | | ↓ | | ∙ | neue Katalysatorlösung |
| Peroxidlösung kontaminiert | | ↑ | | | ↓ | | | frische Peroxidlösung |
| Katalysatorlösung zerfallen | | ↓↓ | ↓ | ↓ | < 30 | < 8,5 | | neue Katalysatorlösung |
| Peroxidlösung zerfallen | | ↓ | ↓ | ↓ | < 30 | | | frische Peroxidlösung |
| Peroxidlösung unvollständig gelöst | | | | | | | ↑ | Peroxidlösung aufschütteln |
| Pipetten defekt - Pipettierfehler | | | | | | | ↑ | Pipetten kontrollieren |
| Injektor arbeitet ungenau | | | | | | | ↑ | Injektor neu einspülen |
| BK I fehlerhaft rekonstituiert | | | ↑↓ | | ↑↓ | ↑↓ | | BK I nochmals rekonstituieren |
| BK II fehlerhaft rekonstituiert | | | | ↑↓ | | ↑↓ | | BK II nochmals rekonstituieren |
| BK I doppelt verwendet | | | | = BKI | | 1 | | Qualitätskontrolle wiederholen |
| BK II doppelt verwendet | | | = BKII | | ↑ | 1 | | Qualitätskontrolle wiederholen |
| BK I und BK II vertauscht | | | ↑ | ↓ | ↑ | < 0,2 | | Qualitätskontrolle wiederholen |

(↑↑ viel zu hoch; ↑ deutlich zu hoch; ↓ deutlich zu niedrig; ↑↓ deutlich vom Erfahrungswert abweichend)